# DEMANDE DE BREVET EUROPEEN

(11) **EP 0 707 004 A1**
(43) Date de publication de la demande: **17.04.1996**
(21) Numéro de dépôt: 95402243.0
(22) Date de dépôt: 09.10.1995
(51) Int. Cl.: C07D 295/18, C07D 295/02, C07D 295/08, A61K 31/495

(54) **Nouveaux dérivés à structure 1-arylalkenyl 4-arylméthyl piperazine, leur procédé de préparation et les compositions pharmaceutiques qui les contiennent**

(30) Priorité: 10.10.1994 FR 9412053
(71) Demandeur: ADIR ET COMPAGNIE, F-92415 Courbevoie Cédex (FR)
(72) Inventeur: Baziard-Mouysset, Geneviève, Fac de Pharmacie, F-31400 Toulouse (FR); Younes, Sallouma, Fac de Pharmacie, F-31400 Toulouse (FR); Labssita, Youssef, Fac de Pharmacie, F-31400 Toulouse (FR); Payard, Marc, F-31130 Balma (FR); Caignard, Daniel-Henri, F-75015 Paris (FR); Renard, Pierre, F-78000 Versailles (FR); Rettori, Marie-Claire, F-92400 Courbevoie (FR)

(57) **Abrégé**

Composés de formule (I) 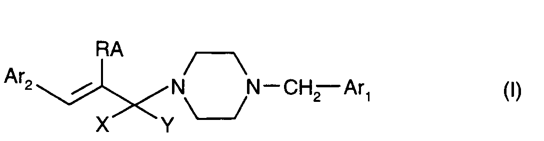
où RA, Ar₁, Ar₂, X et Y sont définis dans la description.

Médicaments.

## Description

La présente invention concerne de nouveaux dérivés à structure 1-arylalkényl 4-arylméthyl pipérazine, leurs procédés de préparation ainsi que les compositions pharmaceutiques qui les contiennent.

La demanderesse a présentement découvert que de tels composés sont de puissants ligands des récepteurs sigma, dépourvus d'affinité pour les autres récepteurs centraux. A ce titre ils peuvent être utilisés dans un certain nombre de troubles du système nerveux central tels qu'anxiété, psychose ou dépression sans les inconvénients majeurs qui accompagnent les thérapeutiques classiques de ces troubles centraux. Parmi ces effets secondaires dont sont dépourvus les composés de l'invention on peut citer somnolence, sédation ou spasmes buccolinguaux, crises oculogyres, ces effets secondaires étant forts génants pour le malade.

La trés haute affinité, pour les récepteurs sigma rend également les composés de l'invention intéressants dans la prévention et le traitement des maladies circulatoires cérébrales, les troubles de la mémoire et la maladie d'Alzheimer, les maladies inflammatoires de type immunitaires telles que l'arthrite et les troubles du péristaltisme intestinal.

Plus particulièrement la présente invention concerne les composés de formule (I) : 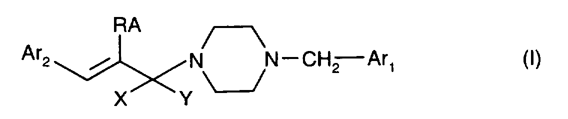
où
Ar₁ et Ar₂ identiques ou différents représentent indépendamment l'un de l'autre un groupe phényle, ou naphtyle ou phényle substitué ou naphtyle substitué
   - X et Y représentent chacun deux atomes d'hydrogène ou forment ensemble un groupement oxo,
   - RA représente un groupement alkyle,
      leurs isomères optiques sous forme pure ou sous forme de mélange et leurs sels d'addition à un acide ou une base pharmaceutiquement acceptable,
   étant entendu que :
   - le terme substitué affectant les substituants phényle et naphtyle signifie que ceux-ci peuvent être substitués par un à trois groupements choisis parmi hydroxy, alkyle, alkoxy, halogène et alkyle substitué par un ou plusieurs halogènes,
   - les termes "alkyle" et "alkoxy" désignent des groupements linéaires ou ramifiés contenant de 1 à 6 atomes de carbone.

De façon préférentielle, l'invention concerne les dérivés de formule (I) pour lesquels :
. RA représente un groupement méthyle et Ar₂ un groupement phényle non substitué de formule (IA) : 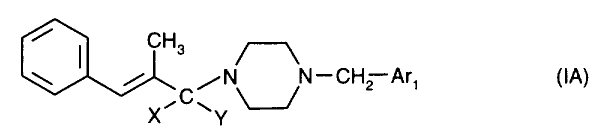 où X, Y et Ar₁ ont la même définition que dans la formule (I)
   leurs isomères optiques sous forme pure ou sous forme de mélange et leurs sels d'addition à un acide ou une base pharmaceutiquement acceptable,

- la 1-(3-phényl-2-méthylprop-2-ènoyl)-4-benzyl pipérazine de formule : 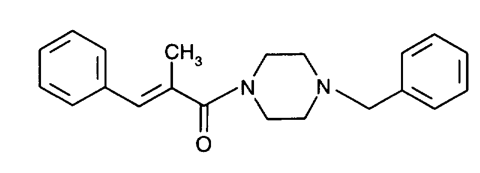 et ses sels d'addition à un acide pharmaceutiquement acceptable,
- la 1-(3-phényl 2-méthylprop-2-ènyl)-4-benzylpipérazine de formule : 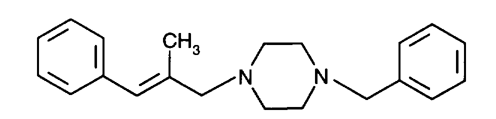 et ses sels d'addition à un acide pharmaceutiquement acceptable
- la 1-(3-phényl-2-méthylprop-2-ènoyl)-4-(4-méthoxybenzyl)pipérazine de formule: 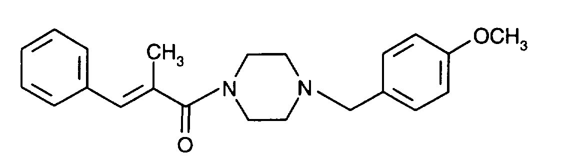 et ses sels d'addition à un acide pharmaceutiquement acceptable,
- la 1-(3-phényl-2-méthylprop-2-ènyl)-4-(4-méthoxybenzyl)pipérazine de formule : 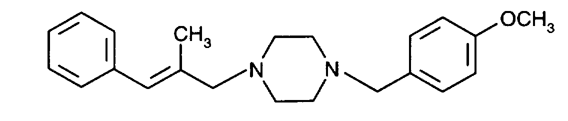 et ses sels d'addition à un acide pharmaceutiquement acceptable,

Parmi les acides pharmaceutiquement acceptables que l'on peut utiliser pour salifier les composés de l'invention on peut citer, à titre d'exemple et de façon non limitative, les acides chlorydrique, bromhydrique, sulfurique, nitrique, oxalique, malique, maléique, succinique, tartrique, méthane sulfonique, camphorique et camphosulfonique.

Parmi les bases pharmaceutiquement acceptables que l'on peut utiliser pour former un sel d'addition avec les composés de l'invention, on peut citer, à titre d'exemples et de façon non limitative, les hydroxydes de sodium, de potassium, de calcium, ou d'aluminium, la triéthylamine, la benzylamine, la diéthanolamine, la tert-butylamine, la dicyclohexylamine, et l'arginine.

L'invention s'étend également au procédé de préparation des composés de formule (I),
caractérisé en ce que
On fait réagir un composé de formule (II) : 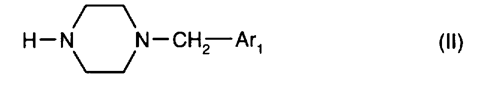 dans laquelle Ar₁ est tel que défini dans la formule (I),
- soit avec un composé de formule (III) : 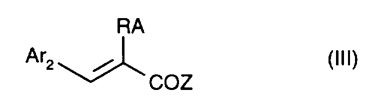 dans laquelle RA et Ar₂ sont tels que défini dans la formule (I) et Z représente un groupement hydroxy ou un atome d'halogène pour obtenir un composé de formule (la) : 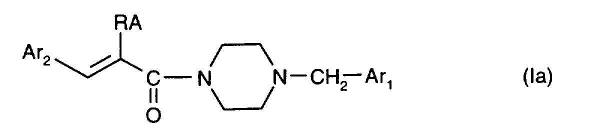 dans laquelle RA, Ar₁ et Ar₂ sont tels que définis précédemment, lequel composé (la) est soumis à une réduction pour donner un composé de formule (Ib) : 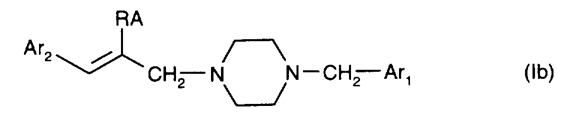 où RA, Ar₁ et Ar₂ ont la même définition que précédemment
- soit avec un composé de formule (IV) : 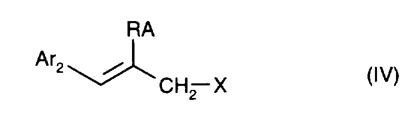 dans laquelle RA et Ar₂ sont tels que définis précédemment et X représente un atome d'halogène, en présence d'un agent alcalin, pour obtenir un composé de formule (lb) tel que défini précédemment,
   les composés de formules (la) et (lb) formant l'ensemble des composés de l'invention qui peuvent, si on le désire, être séparés en leurs différents isomères optiques salifiés avec un acide ou une base pharmaceutiquement acceptable.

Les matières premières utilisées dans le procédé précédemment décrit sont soit commerciales, soit aisément accessibles à l'homme du métier selon des procédés bien connus dans la littérature.

Les chlorures d'acides, s'ils ne sont pas commerciaux, sont obtenus par traitement des acides correspondants avec un agent de chloration comme le chlorure de thionyle.

Les composés de formule (I) possèdent des propriétés pharmacologiques intéressantes.

La très haute affinité des dérivés de l'invention pour les récepteurs σ (sigma) les rend utilisables dans le traitement des troubles liés aux récepteurs sigma, des troubles du système nerveux central, des désordres moteurs tels que les dystonies (Walker JM. Pharmacol. Biochem. Behav. 1990, 36: 151), la dyskinésie tardive (Lindstrom L.H. Acta Psychiatr. Scand. 1988, 77 : 1122), les troubles psychotiques (Chouinard F., Annable L. Psychopharmacology 1984, 84 : 282), et des désordres tels que les dommages liés à l'ischémie, l'insuffisance circulatoire cérébrale, les troubles de mémoire, la maladie d'Alzheimer et l'état de choc (Pontecorvo M.J. Brain Res. Bull. 1991, 26: 461), la régulation des phénomènes immunitaires (Carroll F.l. Med. Chem. Re. 1992, 2 : 3), le traitement des toxicomanies à la cocaïne (Abou - Gharbia M., Academic. Press. lnc. Bristol. J. Ed. Publisher. 1993, 28 : 1), le diagnostic et la localisation des tumeurs (Hudzick T.J., Psychopharmacology. 1992, 108 :115 ; Abou - Gharbia M., Academic. Press. lnc. Bristol. J. Ed. Publisher 1993, 28 : 1), le traitement des vomissements (Hudzick T.J. Eur. J. Pharmacol. 1993, 236 : 279), les maladies inflammatoires d'origine immunitaire comme l'arthrite, l'inflammation bronchopulmonaire et le psoriasis, les pathologies allergiques, l'eczéma, le choc septique, ainsi que les troubles de la motricité intestinale. Pour ce qui concerne le traitement des troubles du système nerveux central, les composés de l'invention sont dépourvus d'affinité pour les récepteurs centraux autres que le récepteur sigma, et sont donc dépourvus des effets secondaires classiquement rencontrés dans ce genre d'activité.

La présente invention a également pour objet les compositions pharmaceutiques contenant les composés de formule (I) ou un de leurs sels d'addition à un acide ou une base pharmaceutiquement acceptable en combinaison avec un ou plusieurs excipients.

Parmi les compositions pharmaceutiques selon l'invention, on pourra citer, plus particulièrement celles qui conviennent pour l'administration orale, parentérale, nasale, per ou transcutanée, rectale perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, et les ampoules buvables ou injectables.

La posologie varie selon l'âge, le sexe et le poids du patient, la voie d'administration, la nature de l'indication thérapeutique, ou des traitements éventuellement associés et s'échelonne entre 0,1 mg et 1 g par 24 heures en 1 ou 2 prises, plus particulièrement 1 à 100 mg, par exemple 1 à 10 mg.

Les exemples suivants illustrent l'invention, mais ne la limitent en aucune façon.

Les spectres de résonance magnétique nucléaire ¹H ont été réalisés en utilisant le TMS (tétraméthylsilane) comme référence interne. Les déplacements chimiques sont exprimés en partie par million (p.p.m). Les spectres infrarouge ont été effectués sous forme de pastille de bromure de potassium renfermant environ 1 % du produit à analyser.

### EXEMPLE 1:

**1-(3-PHENYL-2-METHYLPROP-2-ENOYL)-4-BENZYLPIPERAZINE**

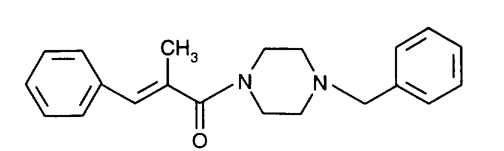

A 0,05 mole de chlorure de l'acide α-méthyl cinnamique E (obtenu par halogénation de l'acide correspondant avec du chlorure de thionyle) en solution dans 100 ml de chlorure de méthylène on ajoute 0,05 mole de benzylpipérazine en solution également dans 100 ml de chlorure de méthylène.On laisse agiter pendant trois heures.
Le précipité est obtenu, filtré et séché et recristallisé dans l'éthanol.

### Caractéristiques physico-chimiques (chlorydrate) :

Rendement : 85 %
C₂₁H₂₅CIN₂O
Poids moléculaire : 356,90
Point de fusion : 202°C
Caractéristiques spectrales
Infra-rouge:
2990 - 3100 cm⁻¹ (ν CH)
1625 cm⁻¹ (ν CON)

Résonance Magnétique Nucléaire ¹H (DMSO-d6)
2,13 ppm (singulet 3CH CH₃)

### EXEMPLE 2 :

**1-(3-PHENYL-2-METHYLPROP-2-ENOYL)-4-(4-METHOXYBENZYL)PIPERAZINE**

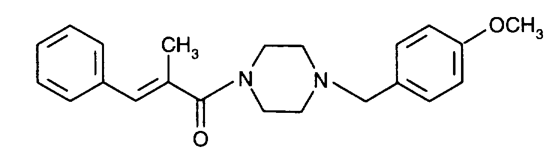

En procédant comme dans l'exemple 1 mais en remplaçant la 1-benzyl pipérazine par la 1-(4-méthoxybenzyl)pipérazine, on obtient le produit du titre.

Caractéristiques physicochimiques (chlorhydrate) :
Rendement : 89%
C₂₂H₂₇ClN₂O₂
Poids moléculaire : 386,93
Point de fusion : 238°C
Caractéristiques spectrales
Infrarouge
3000 - 3100 cm⁻¹ (ν CH)
1620 cm⁻¹ (ν CON)

Résonance Magnétique Nucléaire ¹H(DMSO-d₆)
2,13 ppm (singulet 3H CH₃ ; = C-CH₃)
3,89 ppm (singulet 3H OCH₃)

### EXEMPLE 3 :

**1-(3-PHENYL-2-METHYLPROP-2-ENYL)-4-BENZYLPIPERAZINE**

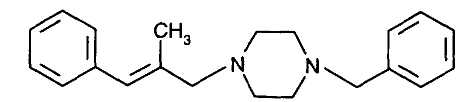

A 0,02 mole de 1-(3-phényl-2-méthylprop-2-ènoyl)-4-benzylpipérazine obtenue dans l'exemple 1 dissoute dans 100 ml de tétrahydrofurane (THF) on ajoute 1 g d'hydrure de lithium aluminium. L'opération est répétée 3 heures plus tard.

Après la fin de la réaction, suivie par chromatographie sur couche mince, l'excès d'hydrure est détruit par 20 ml de méthanol.
Après évaporation à sec, le résidu est repris par 5 ml d'eau et par 100 ml d'acétate d'éthyle. La phase organique est filtrée, évaporée et purifiée par chromatographie sur colonne de gel de silice 60, en utilisant le chlorure de méthylène puis l'acétate d'éthyle comme éluants. On obtient ainsi le produit sous forme de base.
Rendement : 56 %
C₂₁H₂₆N₂
Poids moléculaire : 306
Caractéristiques spectrales
Infrarouge
1605 cm⁻¹ (ν C = C)
Résonance Magnétique Nucléaire ¹H (CDCl₃)
1,89 ppm (singulet 3H CH₃)

### Obtention du dichlorhydrate

Le produit précédemment obtenu est dissous dans 10 ml d'isopropanol chaud. Aprés dissolution, on verse 5 ml d'isopropanol chlorhydrique. Aprés refroidissement, le produit est filtré et séché.
Point moléculaire : 379,38
Point de fusion : 246°C

### EXEMPLE 4 :

**1-(3-PHENYL-2-METHYLPROP-2-ENYL)-4-(4-METHOXYBENZYL)PIPERAZINE**

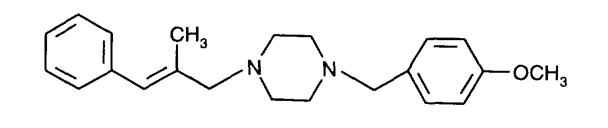

En procédant comme dans l'exemple 3 mais en remplaçant la 1-(3-phényl-2-méthylprop-2-ènoyl)-4-benzylpipérazine par la 1-(3-phényl-2-méthylprop-2-ènoyl)-4-(4-méthoxy benzyl)pipérazine obtenue dans l'exemple 3, on obtient le produit du titre
Rendement : 41 %
C₂₂H₂₈N₂O
Poids moléculaire : 336
Point de fusion : 46°C
Caractéristiques spectrales
Infrarouge
1610 cm⁻¹ (ν C = C)

Résonance Magnétique Nucléaire ¹H (DMSO-d₆)
1,89 ppm (singulet 3H = C-CH₃)
3,79 ppm (singulet 3H OCH₃)

En procédant de même on obtient les composés des exemples 5 à 18 suivants :
5) 1-(3-phényl-2-méthylprop-2-ènoyl)-4-[(napht-1-yl)méthyl]pipérazine
6) 1-(3-phényl-2-méthylprop-2-ènoyl)-4-(4-chlorobenzyl)pipérazine
7) 1 -(3-phényl-2-méthyl-prop-2-ènoyl)-4-(4-hydroxybenzyl)pipérazine
8) 1-(3-phényl-2-méthylprop-2-ènoyl)-4-(2-phényléthyl)pipérazine
9) 1-(3-phényl-2-éthylprop-2-ènoyl)-4-benzylpipérazine
10) 1-(3-napht-1-yl-2-méthylprop-2-ènoyl)-4-benzylpipérazine
11) 1-[3-(3-méthoxyphényl)-2-méthylprop-2-ènoyl]4-benzylpipérazine
12) 1-(3-phényl-2-méthylprop-2-ènyl)-4-[(napht-1-yl)méthyl)]pipérazine
13) 1-(3-phényl-2-méthylprop-2-ènyl)-4-(4-chlorobenzyl)pipérazine
14) 1-(3-phényl-2-méthylprop-2-ènyl)-4-(4-hydroxybenzyl)pipérazine
15) 1-(3-phényl-2-méthylprop-2-ènyl)-4-(2-phényléthyl)pipérazine
16) 1-(3-phényl-2-éthylprop-2-ènyl)-4-benzylpipérazine
17) 1-(3-napht-1-yl-2-méthylprop-2-ènyl)-4-benzylpipérazine
18) 1-[3-(3-méthoxyphényl)-2-méthylprop-2-ènyl]-4-benzylpipérazine
ainsi que leurs sels d'addition à un acide pharmaceutiquement acceptable.

### ETUDE PHARMACOLOGIQUE DES DERIVES DE L'INVENTION

### EXEMPLE A : ETUDE DE LA TOXICITE AIGUE

La toxicité aigue a été appréciée après administration orale à des lots de 8 souris (26 ± 2 grammes ) d'une dose de 100 mg.kg-1 des composés de l'invention. Les animaux ont été observés à intervalles réguliers au cours de la première journée quotidiennement pendant les deux semaines suivant le traitement.
Il apparaît que les composés de l'invention sont totalement atoxiques. lls n'entrainent aucun décès après administration à une dose de 100 mg.kg⁻¹ et on ne constate pas de troubles après administration de cette dose.

### EXEMPLE B : BILAN RECEPTORIEL D'AFFINITE IN VITRO

Les produits sont testés sur chaque récepteur à 5 concentrations différentes (10⁻⁵M, 10⁻⁶M, 10⁻⁷M, 10⁻⁸M, 10⁻⁹M) en triplicata. Lorsque le coefficient de liaison IC₅₀ est inférieur à une concentration de 10⁻⁶M, le Ki est mesuré en utilisant 12 concentrations du produit.
Le tableau ci-après regroupe les récepteurs pour lesquels l'affinité des composés de l'invention a été déterminée, le tissu choisi, la concentration retenue pour déterminer la fraction non spécifique et le radioligand utilisé pour marquer le récepteur.

Les résultats du test ont montré que les composés de l'invention sont de puissants et sélectifs ligands des récepteurs σ. (affinité de l'ordre de 10⁻¹⁰M pour les récepteurs σ, cf Tableau B).

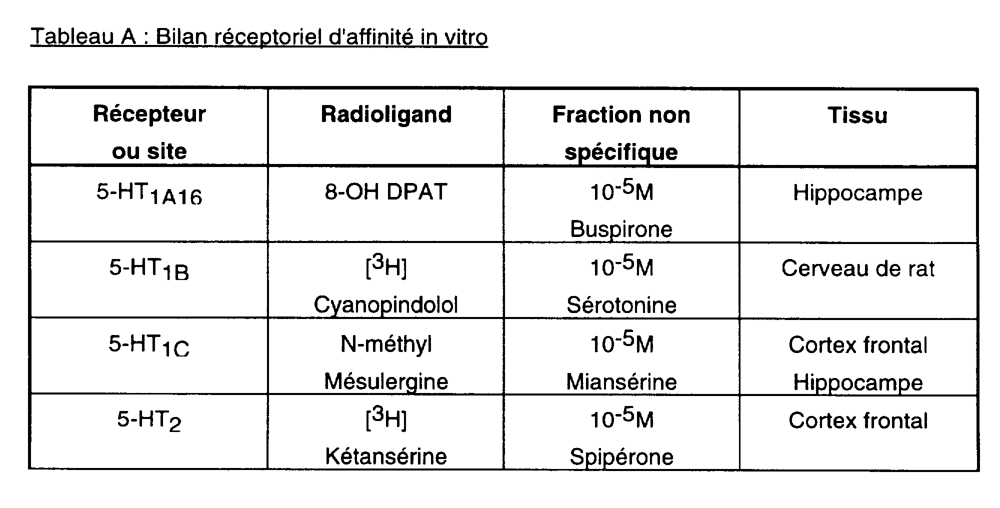

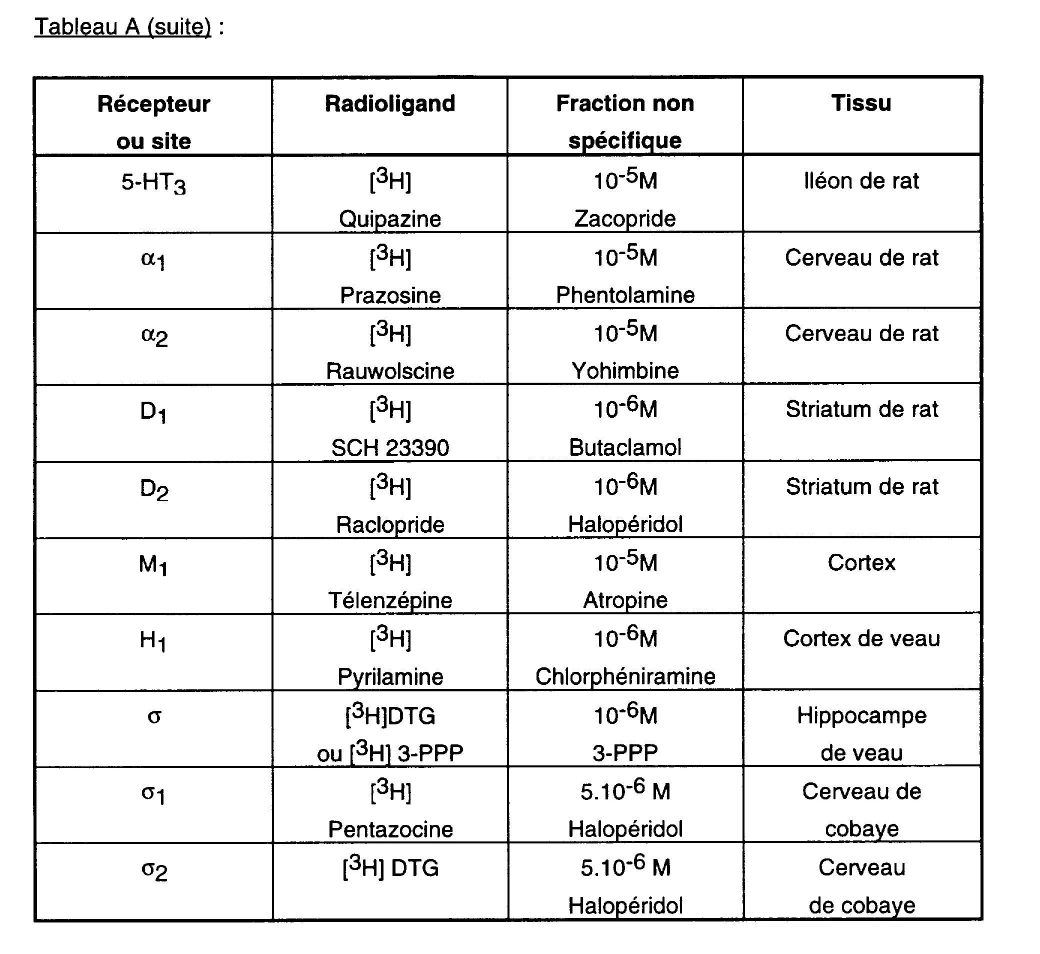

**Tableau B : Résultats du test de liaison aux récepteurs σ**

| **Composé** | **Récepteur** | **Radioligand** | **IC₅₀ (M)** |
|---|---|---|---|
| Exemple 3 | σ | [³H] DTG | 6,9.10⁻¹⁰ |
| Exemple 4 | σ | [³H] DTG | 7,9.10⁻¹⁰ |

### EXEMPLE C: ANTAGONISME DE L'HYPERMOTILITE INDUITE PAR L'AMPHETAMINE

Ce test a été mis au point par COSTALL B. et al (Brain Research 1977, 123 : 89-111).

On injecte à des groupes de 10 souris NMRI-CERJ par voie intrapéritonéale (IP) 4 mg.kg⁻¹ de D-amphétamine juste après le composé à tester (injecé aussi par voie IP), et on place les souris dans un actimètre pendant 30 minutes.

Le nombre d'interruptions de cellules photoélectriques est compté, de même que le comportement stéréotypé.

L'activité des composés testés est exprimée en pourcentage de l'antagonisme de l'hyperactivité induite par l'amphétamine.

Les résultats du test montrent que les composés de l'invention sont de puissants antagonistes de l'hypermobilité induite par l'amphétamine. Ce qui permet de conclure que les composés de l'invention sont de bons candidats pour le traitement des troubles du système nerveux central.

### EXEMPLE D: ANTAGONISME DE L'HYPERACTIVITE INDUITE PAR LA N-ALLYL NORMETAZOCINE

Ce test a été mis au point par Snyder S.H. et al (J. Neuropsychiatry, 1989, 1 : 7-15). La N-allylnormetazocine ((+) SKF 10047) induit un comportement psychotique chez l'homme et est le prototype d'agoniste pour les récepteurs σ.

La mesure de l'hyperactivité induite par ce produit est donc utilisée comme modèle alternatif pour détecter l'activité antipsychotique des composés agissant sur les récepteurs σ.

Un groupe de 12 rats est traité avec le composé à tester avant l'administration sous-cutanée de 50 mg/kg de la N-allylnormetazocine. 30 minutes plus tard, les animaux sont placés dans un actimètre pendant 30 minutes.

L'halopéridol est utilisé comme composé de référence.

Les résultats du test montrent que les composés de l'invention sont de puissants antagonistes de l'hyperactivité induite par la N-allylnormétazocine. Ainsi par exemple le composé de l'exemple 9 à une dose de 32 mg.kg⁻¹ antagonise de 42 % l'hypermotilité induite par la N-allylnormétazocine.

Ce test confirme donc l'intérêt des produits de l'invention pour le traitement des troubles du système nerveux central.

### EXEMPLE E : RECHERCHE D'EFFET CATALEPSIGENE

Ce test a été mis au point par CHERMAT R. et al (J. Pharmacol. 1975, 6 : 493-496).

L'effet catalepsigène est recherché chez le rat par voie intrapéritonéale. 6 groupes de rats Wistar reçoivent une injection des composés de l'invention et sont ensuite testés pour leur activité catalepsigène à 30 minutes d'intervalle. La prochlorpérazine est utilisé comme référence.

Les résultats de ce test révèlent que les composés de l'invention présentent un effet catalepsigène négligeable en comparaison avec la prochlorpérazine dans les mêmes conditions de test. Ce résultat confirme l'absence d'effets secondaires de type extrapyramidaux des produits de l'invention qui pouvait être attendu suite aux résultats de bindings (exemple B).

### EXEMPLE F: ETUDE DE L'ACTIVITE ANTIDEPRESSIVE DES COMPOSES DE L'INVENTION

### PRINCIPE :

L'étude des produits est réalisée sur le modèle du "renoncement appris" qui consiste à induire chez l'animal, par une série d'évènements aversifs incontrôlables, un déficit lors des tâches d'évitement ultérieures.

### PROTOCOLE :

Ce test a été mis au point par Sherman A.D., Sacquitne J.L. et Petty F. (Pharmacol. Biochem. Behav. 1982, 16 : 449-454). Nous utilisons des rats mâles Wistar d'un poids compris entre 180 et 200 grammes. Les animaux sont maintenus à l'animalerie une semaine avant le test, dans des boîtes en plastique, par groupes de 10, à une température ambiante de 21°C ± 1°C, avec libre accès à l'eau et à la nourriture.

Les animaux sont ensuite isolés dans des boîtes de petites dimensions et sont soumis à 60 chocs électriques inévitables (0,8 mA toutes les minutes ± 15 secondes). Un groupe de rats témoins ne reçoit pas de chocs électriques.

La capacité des animaux à réaliser un apprentissage d'évitement (passage d'un compartiment à l'autre, afin d'éviter les chocs électriques) est appréciée 48 heures après et pendant 3 jours consécutifs.

Lors des séances d'apprentissage, les animaux subissent 2 essais par minute pendant 15 minutes. Le nombre d'échecs d'échappement est noté pour chaque rat. Les animaux sont traités (i.p. : 0,5 cm³/100 g) à jeun 6 heures après les chocs inévitables et 4 jours de suite, le matin 30 minutes avant la séance d'apprentissage et le soir entre 18 et 19 h.

Les produits étudiés sont mis en solution dans de l'eau distillée.
Les produits étudiés sont administrés aux doses 0,05 mg.kg⁻¹/jour.

### RESULTATS :

Le test prouve que les produits de l'invention diminuent significativement le nombre d'échecs d'échappement ce qui traduit une forte activité de type antidépressive.

### EXEMPLE G: ETUDE DE L'ACTIVITE ANXIOLYTIQUE - TEST DIT DES CAGES CLAIRE/OBSCURE CHEZ LA SOURIS

### PROTOCOLE :

Ce test a été mis au point par CRAWLEY et al. (1981, Pharmacol. Biochem. Behav. 1981, 15 (5) : 695-699) puis modifié et comportementalement validé.
Il s'agit de deux cages de tailles égale (20 X 20 X 14 cm) en PVC. L'une est fortement éclairée par une lampe de 100 W (lumière "froide"), l'autre est obscurcie. Les deux cages sont séparées l'une de l'autre au moyen d'un petit tunnel opaque (5 X 7 cm). Les souris sont individuellemnt introduites dans la cage obscure, on enregistre, au moyen de claviers reliés à un ordinateur, durant 5 minutes, le temps passé par les animaux dans la cage éclairée ainsi que le nombre des transitions entre la cage obscure et la cage éclairée. Chaque groupe expérimentale comprend au minimum 15 animaux.

### RESULTATS :

L'administration, par voie intrapéritonéale de certains produits de l'invention entraine une augmentation du temps passé par les souris dans la cage éclairée et du nombre des transitions entre la cage obscure et la cage éclairée.

Cette augmentation significative des deux paramètres étudiés montre la remarquable activité anxiolytique des composés de l'invention.

### EXEMPLE H : RECHERCHE D'UNE ACTIVITE DE TYPE ANTIARTHRITIQUE CHEZ LE RAT

### PROTOCOLE :

On utilise des groupes de 5 rats Lewis male ou femelle d'un poids de 130 à 150 g. Une suspension de 0,3 mg de Mycobacterium tuberculosis tués dans 0,1 ml d'huile minérale (Adjuvant de Freund complet, FCA) est administrée dans la région de la patte arrière le Jour 1. Les volumes des pattes arrières sont mesurés par déplacement d'eau les Jours 0, 1, 5, 14 et 18. Les produits à tester sont placés en suspension dans la carboxyméthyl cellulose et administrés oralement pendant 5 jours consécutifs des jours 1 à 5.

### RESULTATS :

On observe après administration des produits de l'invention une diminution significative du volume des pattes arrières dans la phase précoce et dans la phase tardive de l'inflammation (après J 14) ce qui prouve une activité thérapeutique potentielle dans l'arthrite.

### EXEMPLE I : COMPOSITION PHARMACEUTIQUE

Comprimés dosés à 0,1 mg de 1-(3-phényl-2-méthylprop-2-ènyl)-4-benzylpipérazine utilisables dans le traitement des troubles du système nerveux central.

Formule pour 10 000 comprimés :
- 1-(3-phényl-2-méthylprop-2-ènyl)-4-benzylpipérazine 1 g
- Amidon de blé 75g
- Amidon de maïs 75 g
- Lactose 325 g
- Stéarate de magnésium 10 g
- Silice 5 g
- Hydroxypropyl cellulose 10 g

Comprimés dosés à 50 mg de 1-(3-phényl-2-méthylprop-2-ènyl)-4-(4-méthoxybenzyl)pipérazine utilisables dans le traitement de l'arthrite chronique

Formule pour 1000 comprimés
- 1-(3-phényl-2-méthylprop-2-ènyl)-4-(4-méthoxybenzyl)pipérazine 50 g
- Amidon de blé 150 g
- Amidon de maïs 150 g
- Lactose 450 g
- Stéarate de magnésium 10 g
- Silice 5 g
- Hydroxypropyl cellulose 10 g

## Revendications

1. Composés de formule (I) : 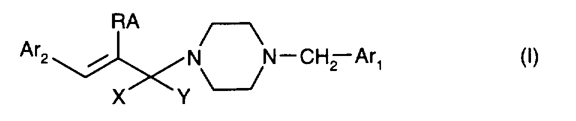 où
Ar₁ et Ar₂ identiques ou différents représentent indépendamment l'un de l'autre un groupe phényle, ou naphtyle ou phényle substitué ou naphtyle substitué
- X et Y représentent chacun deux atomes d'hydrogène ou forment ensemble un groupement oxo,
- RA représente un groupement alkyle,
leurs isomères optiques sous forme pure ou sous forme de mélange et leurs sels d'addition à un acide ou une base pharmaceutiquement acceptable, étant entendu que :
- le terme substitué affectant les substituants phényle et naphtyle signifie que ceux-ci peuvent être substitués par un à trois groupements choisis parmi hydroxy, alkyle, alkoxy, halogène et alkyle substitué par un ou plusieurs halogènes,
- les termes "alkyle" et "alkoxy" désignent des groupements linéaires ou ramifiés contenant de 1 à 6 atomes de carbone.

2. Composés de formule (I) selon la revendication 1 pour lesquels :
. RA représente un groupement méthyle et Ar₂ un groupement phényle non substitué de formule (IA) : 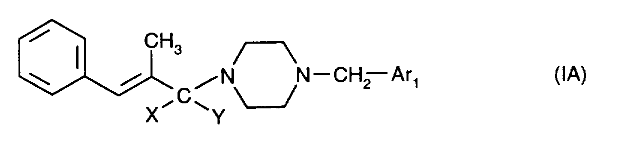 où X, Y et Ar₁ ont la même définition que dans la revendication 1
leurs isomères optiques sous forme pure ou sous forme de mélange et leurs sels d'addition à un acide ou une base pharmaceutiquement acceptable.

3. Composé selon la revendication 1 qui est 1-(3-phényl-2-méthylprop-2-ènoyl)-4-benzyl pipérazine de formule : 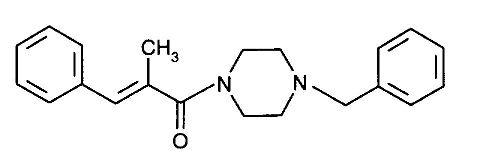 et ses sels d'addition à un acide pharmaceutiquement acceptable.

4. Composé selon la revendication 1 qui est la 1-(3-phényl 2-méthylprop-2-ènyl)-4-benzylpipérazine de formule : 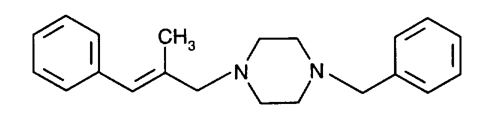 et ses sels d'addition à un acide pharmaceutiquement acceptable.

5. Composé selon la revendiction 1 qui est la 1-(3-phényl-2-méthylprop-2-ènoyl)-4-(4-méthoxybenzyl)pipérazine de formule : 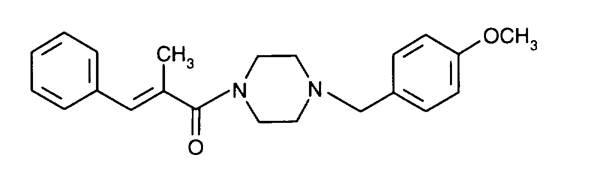 et ses sels d'addition à un acide pharmaceutiquement acceptable.

6. Composé selon la revendication 1 qui est la 1-(3-phényl-2-méthylprop-2-ènyl)-4-(4-méthoxybenzyl)pipérazine de formule : 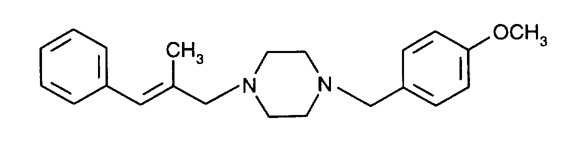 et ses sels d'addition à un acide pharmaceutiquement acceptable.

7. Procédé de préparation des composés de formule (I) selon la revendication 1 caractérisé en ce que
on fait réagir un composé de formule (II) : 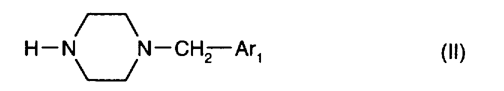 dans laquelle Ar₁ est tel que défini dans la revendication 1,
- soit avec un composé de formule (III) : 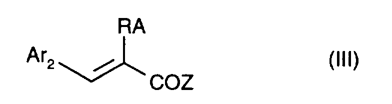 dans laquelle RA et Ar₂ sont tels que défini dans la revendication 1 et Z représente un groupement hydroxy ou un atome d'halogène pour obtenir un composé de formule (la) : 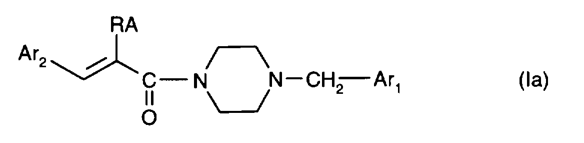 dans laquelle RA, Ar₁ et Ar₂ sont tels que définis précédemment, lequel composé (la) est soumis à une réduction pour donner un composé de formule (Ib) : 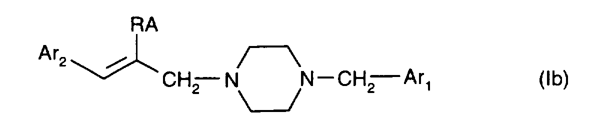 où RA, Ar₁ et Ar₂ ont la même définition que précédemment
- soit avec un composé de formule (IV) : 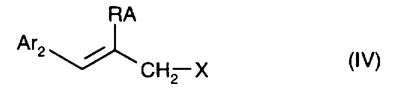 dans laquelle RA, et Ar₂ sont tels que définis précédemment et X représente un atome d'halogène, en présence d'un agent alcalin, pour obtenir un composé de formule (1b) tel que défini précédemment,
les composés de formules (1a) et (1b) formant l'ensemble des composés de l'invention qui peuvent, si on le désire, être séparés en leurs différents isomères optiques salifiés avec un acide ou une base pharmaceutiquement acceptable.

8. Compositions pharmaceutiques contenant les composés de formule (I) selon la revendication 1 ou un de leurs sels d'addition à un acide ou une base pharmaceutiquement acceptable en combinaison avec un ou plusieurs excipients.

9. Compositions pharmaceutiques selon la revendication 8 utiles pour le traitement des troubles du système nerveux central.

10. Compositions pharmaceutiques selon la revendication 8 utiles pour le traitement des maladies inflammatoires d'origine immunitaire.
